# EUROPEAN PATENT SPECIFICATION

(11) **EP 4 438 077 B1**
(45) Date of publication and mention of the grant of the patent: **17.09.2025**
(21) Application number: 23166024.2
(22) Date of filing: 31.03.2023
(51) Int. Cl.: A61M 5/142, A61M 60/00, F04C 2/22, F04C 18/00, A61M 5/14

(54) **MODULAR TROCHOIDAL MEDICAMENT PUMP DEVICE AND DRIVING DEVICE**
MODULARE TROCHOIDENFÖRMIGE MEDIKAMENTENPUMPVORRICHTUNG UND ANTRIEBSVORRICHTUNG
DISPOSITIF DE POMPE À MÉDICAMENT TROCHOÏDALE MODULAIRE ET DISPOSITIF D'ENTRAÎNEMENT

(43) Date of publication of application: 02.10.2024
(73) Proprietor: Medico Invest AG, 9001 St. Gallen (CH)
(72) Inventor: BECHTOLD, Herbert, 78588 Denkingen (DE); GABRIEL, Frédéric, 8052 Zürich (CH); YIP HEUNG WIN, Leanne, Cambridge, CB5 8QD (GB); DE SILVA, Kamaal, Cambridge, CB4 1LQ (GB)
(74) Representative: Maiwald GmbH

(56) References cited:
- US-A1- 2006 271 020
- US-A1- 2019 085 843
- US-A1- 2021 353 854

## Description

### Field of the Invention

The present invention relates to a modular trochoidal medicament pump device, a driving device for the modular trochoidal medicament pump device, a giving set having included said modular trochoidal medicament pump device and a medicament administering system having a modular trochoidal medicament pump device and a driving device therefor, which allows high accuracy, user-friendly application, adaptable area of application, patient-control and safety and an increased sustainability.

US 2019/0085843A1 describes a rotary pump driven medicament delivery device, which however does not have a modular structure, and the pump unit is not designed to be releasably coupled and cannot be separated from the drive. US 2021/0353854A1 describes a drug delivery device, which however is not adapted to be operated with a trochoidal pump unit. US 2006/0271020A1 describes a portable drug delivery device including a detachable and replaceable administration or dosing element, which is based on a peristaltic transportation principle.

### Background of the Invention

Infusion pumps are devices used to deliver drugs into a patient's body. Infusion pumps usually are divided into pumps for stationary, ambulatory, and home use. These pumps are configured differently, resulting in a large number of specific pumps.

Trochoidal pump devices are known for pumping applications, e.g., from US 4,137,024 which describes a rotary piston mechanism such as compressors and expansion engines, and from JP-S-62-17322, which describes a rotary piston type compressor. Trochoidal pump devices are also known for pumping applications in refrigerators, as described e.g., in US 6,520,754, which describes a compressor for a refrigerator for household applications.

A trochoidal pump device for medical applications is also known, e.g., from WO 2019/057570, which describes a rotary pump for use in a medicament delivery device.

Over the known prior art it can be considered as an object to provide a modular trochoidal medicament pump device and a driving device therefor, a giving set having included said modular trochoidal medicament pump device and a medicament administering system with high accuracy, user-friendly application, adaptable area of application, patient-control and safety and an increased sustainability.

### Summary of the Invention

The invention is defined according to the appended claims.

The present invention provides a modular trochoidal medicament pump device, a driving device therefor, a giving set including said modular trochoidal medicament delivery device, and an administering system according to the independent claims, whereas further embodiments are incorporated in the dependent claims.

There is provided a medicament pump driving device for controlled driving of a medicament trochoidal medicament pump device having a pump body and a pump rotor rotatable therein with a driving input interface; the medicament pump driving device comprising a housing, a receptacle bayed in said housing for releasable receiving of said pump body of said trochoidal pump device to be connected to the medicament pump driving device, an output driving interface for releasable receiving said input driving interface of said trochoidal medicament pump device for driving said pump rotor, a drive unit arranged within the housing for rotatable driving via the output driving interface said pump rotor of said trochoidal medicament pump device, a control unit for controlling the drive unit to drive the output driving interface based on a provided dosing signal.

Thus, a medicament pump driving device can be provided as an infusion pump device, which is reusable. The receptacle may be adapted in shape to the modular trochoidal medicament pump device in order to have a keying, which may avoid misuse of a wrong modular trochoidal medicament pump device with the medicament pump driving device. The medicament pump driving device may be adaptable and be used as a basic module. The medicament pump driving device may be combined with a disposable modular trochoidal medicament pump device as a pump module, which may be releasable or fixed connected to a primary medication container or reservoir. The modular trochoidal medicament pump device may be designed as a single use device or a limited number of use device. The medicament pump driving device as a basic module may contain all reusable components such as a motor-gear unit, control unit, display unit, energy source and the safety features, sensors, patient identification unit, drug identification unit, user safety features, and locking mechanism and logics. The medicament pump driving device may be operated with different modular trochoidal medicament pump devices, which may be selected upon the application, the liquid to be administered, the safety requirements, the dosing rate, and the like. According to an embodiment, the output driving interface comprises a coupling having an engaging geometry for receiving a corresponding counter coupling of said input driving interface for coupling in a number of defined rotational positions of said pump rotor of said trochoidal medical pump device.

Thus, a defined rotational position of a pump rotor over a pump body and housing may be recognized. The rotational position information may be transferred by mutual engaging geometry between driving unit represented by a driving engine motor and a gearing. In case the repeated symmetry of the rotor geometry within the trochoidal medical pump device corresponds to the number of defined equidistant rotational positions, the rotational position of the output driving interface allows a conclusion on the rotational position of the rotor within the pump housing.

According to an embodiment, the medicament pump driving device comprises a position receiving sensor for receiving a rotational position of said pump rotor.

Thus, regardless of the rotational position of the output driving interface the rotational position of the pump rotor within the pump housing or pump chamber may be determined. The position receiving sensor senses a corresponding information provided by a position signaler on, e.g., the trochoidal medicament pump device. As a trochoidal pump has a non-linear driving characteristic, the rotational position of the rotor within the housing may be helpful for determination and control of the dosing rate upon rotation of the output driving interface and thus the rotor in the pump for providing an exact desired dose rate. This may also include a position sensing of a driving shaft, as long as the relative rotational position of the driving shaft and the pump rotor is known.

According to an embodiment, the position receiving sensor is adapted to receive at least one of an optical signal, an inductive signal, a resistive signal, and a capacitive signal representing a rotational position of said pump rotor within said pump body of said trochoidal medicament pump device.

Thus, a position sensor for receiving the position of the pump rotor can be provided, which may receive the information contact based or contactless. The sensor may be path sensor reading an optical pattern, e.g., by a laser or a reflector pattern, an inductive or capacitive coupling or a resistive pattern on said trochoidal medicament pump device. As an alternative the sensor may also be a receiver for an actively transmitted position information from the trochoidal medicament pump device. It should be noted that also other sensing principles may be used, e.g., a hall effect sensor, a mechanical triggering system, e.g., a cam with a micro-switch or the like.

According to an embodiment, the medicament pump driving device comprises a locking mechanism being adapted for locking said trochoidal medicament pump device into the pump driving device during a driving operation.

Thus, it can be avoided that during driving operation of the medicament pump driving device the trochoidal medicament pump device is unintentionally removed from the medicament pump driving device. This may be realized by a mechanical lock or by a logical lock, which may lock a release button. The driving operation may include driving periods and pausing periods, or delay times after administering, which are to be maintained for medical reason.

According to an embodiment, the medicament pump driving device comprises an access cover being adapted for securely covering said trochoidal medicament pump device and releasing a driving operation.

Thus, the access to the trochoidal medicament pump device may be limited against unintentional removal of the trochoidal medicament pump device or other impact onto the trochoidal medicament pump device. The access cover may hold said trochoidal medicament pump device in place. The access cover may also have a locking mechanism and a logic, which may shout down drive control trigger upon opening thereof. At least one of the trochoidal medicament pump device locking mechanism and the access cover locking mechanism are releasable only upon authorization, e.g., releasable only upon permission of e.g., medical personal and/or the patient, in order to avoid misuse. Upon activation of a de-locking, a drive control being adapted to at least one of moving the drive unit to a non-tensioned state with respect to said coupled trochoidal medicament pump device, releasing the trochoidal medicament pump device locking mechanism and releasing the access cover locking mechanism. This can also be coordinated by the control of the medicament pump driving device by first bringing the drive unit to a non-tensioned state with respect to said coupled trochoidal medicament pump device, then releasing the trochoidal medicament pump device locking mechanism and then releasing the access cover locking mechanism, so that the modular trochoidal medicament pump device can be removed without any unintended consequences.

According to an embodiment, the medicament pump driving device comprises a rechargeable energy storage being adapted for providing operation energy for the drive unit and the control unit.

Thus, the medicament pump driving device may be provided as a wearable unit which makes the patient more independent. The energy storage may be an exchangeable battery for a longer runtime, or a rechargeable battery, exchangeable or fixed mounted, for sustainability reason.

According to an embodiment, the medicament pump driving device comprises a wireless charging interface being operatively connected to the rechargeable energy storage.

Thus, an energy storage, like a rechargeable battery may be charged upon putting the device to the vicinity of a wireless operating charging device. The medicament pump driving device may be designed as a hermetically sealed device, which may be operated also under difficult environmental conditions and may be subject of an easy disinfection or sterilization.

According to an embodiment, the medicament pump driving device comprises a dosing data interface being operatively connected to the control unit for providing dosing data to and from the medicament pump driving device.

Thus, the intended dosing rate may be transmitted, e.g., wireless, to the control unit of the medicament pump driving device. Also actually administered doses rates may be transmitted from the medicament pump driving device to e.g., an information system, e.g., a hospital information system. Transfer of dosage information to the control unit for controlling dosage may be carried out as well as transfer of dosage information from the control unit for monitoring a dosage. Further, an internal storage for storing dosing data may be provided for buffering data to be transmitted or archiving data. A wireless transmission of data to and from the medicament pump driving device allows a hermetic sealing of the medicament pump driving device for disinfection and sterilization purposes.

According to an embodiment, the medicament pump driving device comprises a hermetically closed user interface.

Thus, also the user interaction can be carried out while maintaining the hermetically closed medicament pump driving device. For this purpose, the medicament pump driving device may be provided e.g., with a touch screen user interface and capacitive and/or inductive buttons.

According to an embodiment, the medicament pump driving device comprises a tag reader being adapted for reading a tag on a medicament reservoir being fixedly connected to said trochoidal medicament pump device.

Thus, the medicament pump driving device may read out a corresponding tag on the trochoidal medicament pump device or a medicament contained thereof, which may allow identification of the liquid stored in the container, and/or the type of the trochoidal medicament pump device, so that the control unit of the medicament pump driving device may control the drive unit of the medicament pump driving device accordingly.

According to an embodiment, the medicament pump driving device comprises a patient identification reader being adapted for reading a patient identifier and releasing the operation of the drive unit upon a patient identification.

Thus, the medicament pump driving device may read out a corresponding identifier on the patient, which may be tag or a label or even a fingerprint or an iris of the patient, which may allow identification of the patient to check the correct allocation of an administered dose, type of medicament or the like. The medicament pump driving device may correlate a read-out patient identification and a read-out identification of the trochoidal medicament pump device or liquid in a container. Thus, a mismatch of a medicament and a patient, and/or the type of the trochoidal medicament pump device may be avoided, so that the control unit of the medicament pump driving device may control the drive unit of the medicament pump driving device accordingly. This may significantly increase safety in application of medicament, in particular for vulnerable patients.

According to an embodiment, the medicament pump driving device comprises a healthcare professional identification reader being adapted for reading a healthcare professional identifier and monitoring the operation of the drive unit upon a healthcare professional identification.

Thus, the medicament pump driving device may read out a corresponding identifier on the healthcare professional, which may be tag or a label or even a fingerprint or an iris of the healthcare professional, which may allow identification of the healthcare professional to monitor the handling and operation upon identification of a healthcare professional or the like. The medicament pump driving device may correlate a read-out healthcare professional identification and a read-out identification of the trochoidal medicament pump device or liquid in a container. Thus, an erroneous operation selected by a healthcare professional or the like of the trochoidal medicament pump device may be avoided or at least be tracked. This may significantly increase safety in application of medicament, in particular for vulnerable patients.

According to an embodiment, there is provided a trochoidal medicament pump device to be coupled to a medicament pump driving device, e.g., as described above, the trochoidal medicament pump device comprises a pump body with an outer geometry being adapted for being received in an receptacle bayed in said housing of said medicament pump driving device, an in-port for entering a medicament to be administered, an out port for administering a medicament, a pump rotor having a convex triangular cylinder shape and having an eccentric trajectory upon rotation within the pump body, an input driving interface coupled to the pump rotor and being adapted for releasable coupling to a corresponding output driving interface of said medicament pump driving device, wherein the pump rotor has three equidistant apexes, each two thereof are connected with a convex curved line, wherein the pump body has a pump chamber, wherein the pump chamber has a first lobe forming a first inner wall portion of a pump volume of the pump chamber with a first inlet in fluid communication with the in-port and a first outlet in fluid communication with the out-port with the pump rotor eccentrically rotating so that at least three distinct portions of the rotor are in permanent sealing contact with the first inner wall portion of the first lobe thus forming upon rotation a first suction volume in fluid communication to the first inlet and a first expelling volume in fluid communication to the first outlet.

Thus, a modular trochoidal medicament pump device may be provided which may exchangeable coupled to the medicament pump driving device, e.g., like a medicament pump driving device as describe above. The convex triangular cylinder-shaped rotor in combination with the trochoidal pump chamber allows a reliable pumping process. A convex triangular cylinder shape is to be understood as a face of a convex triangle which is expanded into the orthogonal direction in order to form a geometrical body. The pump has an inverse operation principle to a so-called Wankel motor. The dose rate may be exactly set with such a pump. The modular trochoidal medicament pump device as a pump module is designed as a self-sucking pump allowing sucking liquid from a reservoir below the level of the trochoidal medicament pump device. The trochoidal medicament pump device may also operate as a bi-directional pump, which offers the option of connecting different primary packaging. As the pump module may work bidirectional, it is able to also fill fluid cassettes that can be coupled to the trochoidal medicament pump device as base module without having gas or air in the reservoir which may deteriorate the medicament liquid. The outer geometry of the pump body may be provided with an orthogonal cross-section of an outer shape being designed to be securely received in a corresponding receptacle or bay of said medicament pump driving device. The eccentric operation of the rotor within the pump chamber may be achieved with a planetary gear or an eccentric shaft, so that the input driving interface remains on the axis, but the rotor rotates eccentrically.

According to an embodiment, the pump chamber has a second lobe symmetrical to the first lobe, a second suction volume and a second expelling volume wherein the second lobe has formed therein a pressure-bypass between the second suction volume and a second expelling volume. This bypass may be realized in that the second inner wall portion has in a circumferential direction at least section-wise a notch formed therein forming upon rotation of the pump rotor a pressure compensation bypass between the second expelling volume and the second suction volume.

Thus, upon use of only one lobe for pumping, the pressure build-up and under-pressure may be compensated. The purpose of the bypass in the second chamber area is to reduce the drive torque, and sealing defects driven by the generated air pressure in the second chamber. As the pressure upon rotation in the second suction chamber and the second expelling chamber are compensated, no second inlet and no second outlet have to be provided for the pressure compensation. Thus, the pump chamber may be sealed over the environment, which reduced impurities from outside.

According to an embodiment the pump chamber has a second lobe forming a second inner wall portion of a pump volume of the pump chamber with a second inlet in fluid communication with the in-port and a second outlet in fluid communication with the out-port with the pump rotor eccentrically rotating so that at least three distinct portions of the rotor are in permanent sealing contact with the second inner wall portion of the second lobe thus forming upon rotation a second suction volume in fluid communication to the second inlet and a second expelling volume in fluid communication to the second outlet.

Thus, a symmetrical pump geometry can be provided which provides two parallel operating pump sequences, one on the first lobe and the other on the second lobe. As the rotor is triangular and the pump chamber provides two lobes, the phase shift between the sucking an expelling characteristic of the first lobe is 180° over the sucking an expelling characteristic of the second lobe. When coupling both pump sequences together, the one thereof has a maximum expelling when the outlet is in the middle between two apexes, while the other one thereof has a minimum expelling when the third apex passes the outlet. A check valve at the outlet of each expelling chamber may avoid reflow of fluid into the expelling chamber. The same may apply for an inlet, and a check valve at the inlet of each suction chamber may avoid flow of fluid out of the suction chamber. It should be noted that the joint between the first lobe and the second lobe, i.e. where the first inner wall portion and the second inner wall portion transit into each other, is to be considered as belonging to both, the first lobe and the second lobe, and to both, the first inner wall portion and the second inner wall portion. It should also be noted, that the at least three distinct portions of the rotor in permanent sealing contact for the first lobe and the at least three distinct portions of the rotor in permanent sealing contact for the second lobe may share a portion, which may be a contact of the convex curved surface with the transit from the first lobe to the second lobe.

According to an embodiment, the second inlet is in fluid communication with the in-port and the second outlet is in fluid communication with the out-port.

Thus, not only first inlet, but also the second inlet can be connected to a common in-port, and also both, the first and second outlet is in fluid communication with the out-port so that the trochoidal medicament pump device can be operated with both lobes in parallel while having a single in-port and a single out-port, wherein the coupling of the parallel operated lobes and their inlets and outlets is established internally.

According to an embodiment, the trochoidal medicament pump device comprises a position signaler for signaling a rotational position of the pump rotor within the pump body.

Thus, the trochoidal medicament pump device may signal the rotational position of the rotor with respect to the pump body. The medicament pump driving device may use this positional information for controlling the dose rate. Regardless of the rotational position of the output driving interface the rotational position of the pump rotor within the pump housing or pump chamber may be determined. The position signaler provides information on the rotational position of the rotor in the trochoidal medicament pump device. As a trochoidal pump has a non-linear driving characteristic, the rotational position of the rotor within the housing may be helpful for determination and control of the dosing rate upon rotation of the output driving interface and thus the rotor in the pump for providing an exact desired dose rate.

According to an embodiment, the signaler is adapted to signal at least one of an optical signal, an inductive signal, a resistive signal, a capacitive signal representing a rotational position of the pump rotor within said pump body.

Thus, a position signaler for providing the position of the pump rotor can be provided, which may provide the information contact based or contactless. The signaler may be path signaler providing an optical pattern, which can be read by a e.g., a laser or a reflector pattern, an inductive or capacitive coupling or a resistive pattern on said trochoidal medicament pump device. As an alternative the signaler may also be an active sender for a position information from the trochoidal medicament pump device. It should be noted that also other signaler principles may be used, e.g., a hall effect signaler, a mechanical triggering signaler, e.g., a cam with a micro-switch or the like.

According to an embodiment, the trochoidal medicament pump device comprises a locking receptacle being adapted for receiving a locking mechanism of said medicament pump driving device during a driving operation.

Thus, the trochoidal medicament pump device may be reliably locked with respect to the medicament pump driving device in order to avoid unintentional removal and separation thereof.

According to an embodiment, there is provided a giving set for providing a medicament from a reservoir to a patient, the giving set comprises a trochoidal medicament pump as describe above, a first fluid conduit being with one end fixedly connected to the out-port and with the other end fixedly connected to a Luer-lock.

Thus, the giving set can be provided with a medicament pump device without the need for additional joints between the pump device and the conduits. This may avoid impurities and keeps the possible number of connecting joints low. Further, it can be avoided that the medical pump device is unintentionally re-used, as the giving set definitively will not be re-used and disposed and so also the medical pump device. This makes the entire operation of the giving set safer and compliant with current practices for said operation.

According to an embodiment the first fluid conduit has an upstream section and a flexible downstream section, wherein the flexible downstream section is more flexible than the upstream section.

Thus, the giving set may stay more reliable connected to the patient. More flexible means that the more flexible section has a lower bending moment, in that the force required to bend to a certain extend along a given length is lower, in other words, more stable regarding bending. The flexible downstream section close to the patient allows compensation of a movement of the medicament pump device and unintentional separation of the giving set from the patient.

According to an embodiment, the first fluid conduit is made of a flexible tube of substantially constant diameter and wall thickness, wherein the flexible downstream section is formed by a pre-formed helical winding of the flexible tube, the geometry thereof providing higher flexibility than a non-helical portion.

Thus, it is possible to not only compensate a lateral, i.e. orthogonal to the extension of the conduit, movement of the medical pump device over the patient, but also a longitudinal, i.e. along the extension of the conduit, movement of the medical pump device over the patient. It should be noted that this may also include an embodiment, which has for the upstream portion a helical winding with low diameter windings, i.e. lower flexibility, and for the downstream portion a helical winding with large diameter windings, i.e. a higher flexibility.

According to an embodiment the giving set further comprises a second fluid conduit being with one end fixedly connected to the in-port and with the other end fixedly connected to a connector being adapted for being releasable connected to a fluid reservoir.

Thus, the giving set can be provided with a medicament pump device without the need for additional joints between the pump device and the conduits to the reservoir. This may avoid impurities and keeps the possible number of connecting joints low. Further, it can be avoided that the medical pump device is unintentionally re-used, as the giving set definitively will not be re-used and disposed and so also the medical pump device. This makes the entire operation of the giving set safer. It should be noted that the giving set may also a fixedly connected dripping volume between the reservoir and the medicament pump device.

According to an embodiment, the giving set further comprises a second fluid conduit being with one end fixedly connected to the in-port and with the other end fixedly connected to a pre-filled fluid reservoir.

Thus, a safe given set may be provided, which has a minimal number of connections and possible entering points for impurities. The medicament pump device may be adapted to the fluid in the pre-filled reservoir, so that the viscosity of the fluid matches the design of the medicament pump device. Any re-use can be avoided, which is of particular relevance for sensitive medicaments and critical diseases. The prefilled reservoir may include a predefined composition of medicament. A misalignment of medicament, in particular fluid and pump may be avoided. The reservoir may by formed by a cassette having two layers, one outer layer being a hard cover, e.g., transparent plastic case, and one inner layer being inside of the hard cover or case. The inner layer may be flexible, e.g., a bar, in particular a transparent bag and thus of a varying volume, to avoid ventilation and entering air from outside upon taking out a liquid medicament out of the bag.

According to an embodiment, the prefilled reservoir is gas volume free prefilled with a liquid.

Thus, the suction of gas can be avoided, and it can be guaranteed that regardless of the filling level of the reservoir always a fluid is delivered upon operation. As the trochoidal medicament pump device due to its construction is able to also suck against a certain height, the reservoir can also be disposed lower than the trochoidal medicament pump device. This makes it much easier to handle for a patient, in particular if the medicament pump device and the reservoir are used as a mobile delivery system which is carried by a patient and allows moving around.

According to an embodiment the giving set further comprises a tag representative for the liquid in the pre-filled reservoir and being adapted to be read out by a tag reader of a medicament pump driving device.

Thus, the operation of the giving set with the trochoidal medicament pump device may be adapted to the type of fluid or liquid in the reservoir. Some liquids may have particular properties, which allow a defined driving dynamic, which can be setup based on the read-out tag, so that not only the medicament pump may be adapted to the liquid, but also the operation, e.g., the revolutions or change rate of revolutions, of the medicament pump device. This may of particular relevance if the liquid is a or includes a non-Newtonian fluid.

According to an embodiment, there is provided a medicament administering system comprising a medicament pump driving device as described above and a trochoidal medicament pump device as described above, wherein the trochoidal medicament pump device with its pump body is bayed in a receptacle in the housing of the medicament pump driving device for releasable receiving the pump body, wherein an outer shape of the pump body matches the receptacle in that the pump body upon reception within the receptacle is rotationally fixed to the housing of the medicament pump driving device.

Thus, also a combination of the trochoidal medicament pump device and a driving device therefor is provided, which allows exchanging of the trochoidal medicament pump device over the driving device, however with a reliable connection between the trochoidal medicament pump device and the driving device upon coupling. The trochoidal medicament pump device may be exchanged upon delivery of another fluid or liquid, so that the pump may be adapted to the liquid to be administered. The trochoidal medicament pump device may also be exchanged after use, in particular if a single use is intended. The driving device may be reused. The trochoidal medicament pump device provides a high accuracy for the delivery rate as it may be reproducibly operated for a medicament administering. This may provide a higher accuracy compared to peristaltic pumps.

It should be noted that the above-described embodiments may also be combined and in a combined form provide a synergetic technical effect and synergetic benefits which go beyond the sum of the single technical effects and benefits.

### Brief Description of the Figures

The invention will be described by way of the following drawings:
- Figure 1:: illustrates a schematic build-up in a top view of a trochoidal medicament pump device according to an exemplary embodiment.
- Figure 2a:: illustrates medicament pump driving device with a not yet connected trochoidal medicament pump device an according to an exemplary embodiment.
- Figure 2b:: illustrates medicament pump driving device with an already connected trochoidal medicament pump device an according to an exemplary embodiment.
- Figure 3a:: illustrates medicament pump driving device with removed cover part with a not yet connected trochoidal medicament pump device an according to an exemplary embodiment.
- Figure 3b:: illustrates medicament pump driving device with removed cover part and an almost connected trochoidal medicament pump device an according to an exemplary embodiment.
- Figure 4:: illustrates a top view of a trochoidal medicament pump device with removed cover according to an exemplary embodiment.
- Figure 5:: illustrates an exploded view of a trochoidal medicament pump device without cover according to another exemplary embodiment.
- Figure 6:: illustrates a schematic build-up in a top view of a trochoidal medicament pump device according to another exemplary embodiment.
- Figure 7:: illustrates a trochoidal medicament pump device with a connected liquid/fluid reservoir and a trochoidal medicament pump device according to an exemplary embodiment.
- Figure 8:: illustrates trochoidal medicament pump device with a connected liquid/fluid reservoir and a trochoidal medicament pump device connected to driving device according to an exemplary embodiment.
- Figure 9:: illustrates giving set with a fluid conduit to be connected to a patient and a fluid conduit to be connected to a liquid reservoir according to an exemplary embodiment.

It should be noted that same or similar reference numerals illustrate same or similar components. Along these Figures exemplary embodiments of the invention will be described as follows.

### Detailed Description of Exemplary Embodiments

The invention will be described along exemplary embodiments, which are illustrated in the above references figures and will be described in detail in the following.

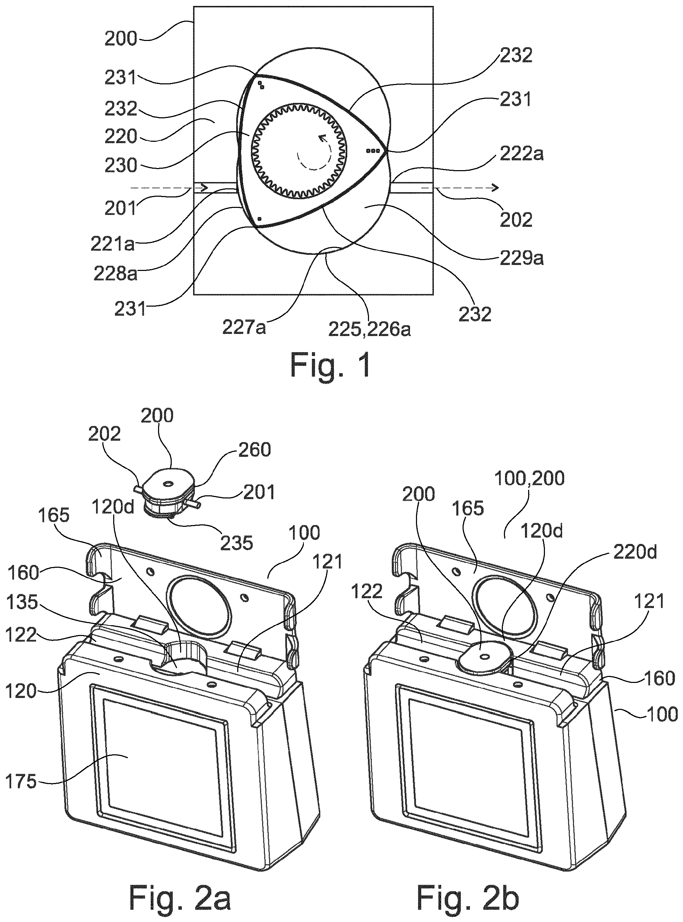

Figure 1 illustrates a schematic build-up in a top view of a trochoidal medicament pump device according to an exemplary embodiment. The trochoidal medicament pump device 200 has an in-port 201 and an out-port 202. The in-port 201 receives the fluid or liquid to be pumped and the out-port 202 delivers the fluid or liquid to be delivered. The in-port and the out-port may have releasable couplings or may be fixedly connected to conduits or the like. The trochoidal medicament pump device 200 has a pump body 220, which may have a housing 220a and a cover 220b. The housing 220a and the cover 220b define within the pump body 220 a pump chamber 225. The pump chamber 225 has rotating therein a pump rotor 230. The pump rotor 230 is of a triangular shape when seen from top. Seen from top the triangular shape has three apexes 231 and between each two apexes a convex curved line 232. When seen as a three-dimensional object, the apexes 231 are apex lines 231a extending parallel to the rotational axis of the pump rotor 230, and the convex curved lines 232 are convex curved surfaces 232a, extending between the apex lines 231a. The apex lines 231a and the convex curved lines 232a are in contact with wall portions 227a, 227b of the pump chamber 225. The apex lines 231a are always in contact with one of the wall portions 227a, 227b. The pump chamber 225 has two lobes 226a, 226b. Within the pump chamber 225 the pump rotor 230 rotates, so that the contact between the pump rotor 230, in particular its apex lines 231a and also the convex curved surfaces 232a, and the wall sections 227a, 227b of the lobes 226a, 226b form chambers, which rotate with the eccentrically rotating pump rotor 230 and successively are brought into communication with an inlet 221a and an outlet 222a. When in connection with the, or one of the inlets 221a, the chamber operates as a suction chamber 228a. As the chamber upon rotation successively enlarges its volume, the chamber sucks a fluid or liquid from the in-port 201. Upon rotation, the suction chamber rotates until it leaves the inlet 221a. Afterwards, the chamber gets into communication with an outlet 222a and becomes an expelling chamber 229a. Upon further rotation, the expelling chamber 229a successively decreases its volume and thus expels the fluid through the output 222a until the volume arrives at its minimum, which may be close to zero. At the same time, the following geometry of the rotor 230 proceeds likewise, so that the next chamber becomes the expelling chamber 229a and expels the next fluid amount upon rotation of the pump rotor 230.

Figure 2a illustrates a medicament pump driving device 100 with a trochoidal medicament pump device 200, which is not yet received in a receptacle 120d of the medicament pump driving device 100. The medicament pump driving device 100 has a housing 120 and within the housing 120 a receptacle 120d for receiving the trochoidal medicament pump device 200. The receptacle 120d has a shape, which corresponds to the outer shape 220d of the trochoidal medicament pump device 200, in order to securely receive the trochoidal medicament pump device 200. This may avoid rotation of the trochoidal medicament pump device 200, so that the rotor 230 within the pump body 220 properly rotates and delivers the fluid to be pumped. Upon reception of the trochoidal medicament pump device 200 within the receptacle 120d, an input driving interface 235, e.g., a driving pinion of the trochoidal medicament pump device 200 engages a corresponding output driving interface 135 of the medicament pump driving device 100. Thus, the trochoidal medicament pump device 200, in particular its rotor 230 can be driven by the medicament pump driving device 100. The housing 120 may also have a canal 121 for receiving a fluid conduit (not illustrated here) being or to be connected to the in-port 201 of the trochoidal medicament pump device 200. The housing 120 may also have a canal 122 for receiving a fluid conduit (not illustrated here) being or to be connected to the out-port 202 of the trochoidal medicament pump device 200. The receptacle 120d, and/or the canals 121, 122 may be covered by a lid or access cover 165 in order to protect the trochoidal medicament pump device 100 from dirt, unauthorized or unintended access. The trochoidal medicament pump device 200 may be locked to the medicament pump driving device 100. For this purpose, a locking mechanism 160 or a locking logic may be provided, which mechanism 160 may engage into a corresponding locking geometry 260 of the trochoidal medicament pump device 200. The locking mechanism 160 may also be realized by locking the access cover 165. The medicament pump driving device may also have a user interface 175 for user input and user output. This user interface 175 may e.g., a touch screen, or the like, so that the entire housing 120 may be sealed over the environment for cleaning, disinfection and/or sterilization purposes.

Figure 2b illustrates the medicament pump driving device 100 having received in its receptacle 120d the trochoidal medicament pump device 200, which matches with its outer shape 220d the inner shape of the receptacle 120d. In this embodiment, the trochoidal medicament pump device 200 cannot rotate within the receptacle 120d. Fluid conduits are not illustrated here, but may be received within the canals 121, 122. When closing the (here still open) lid or access cover 160, the trochoidal medicament pump device 200 and the fluid conduits at least partially are covered and protected. The access cover 165 may also be provided with a sensor and an alert, so that opening of the access cover will initiate an alert, so that the personal or the patient may take care. The access cover 165 may have a window, which may allow inspecting the trochoidal medicament pump device 200, and its operation. In particular, in case the trochoidal medicament pump device 200 is provided with a transparent case or cover, the pump rotor 230 within the trochoidal medicament pump device 200 may be inspected and monitored through the window of the access cover 165.

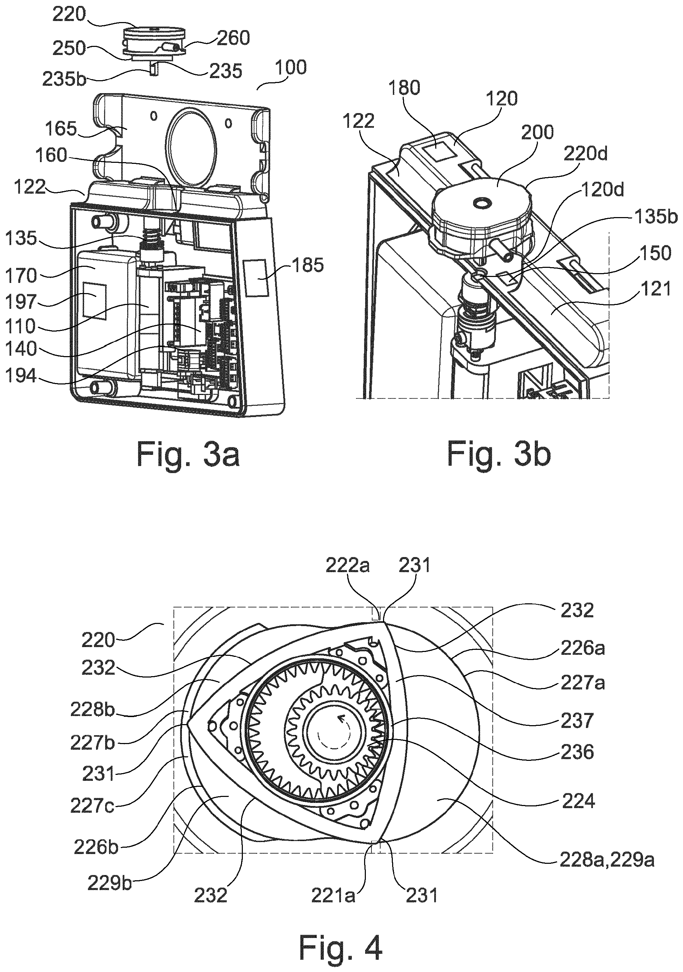

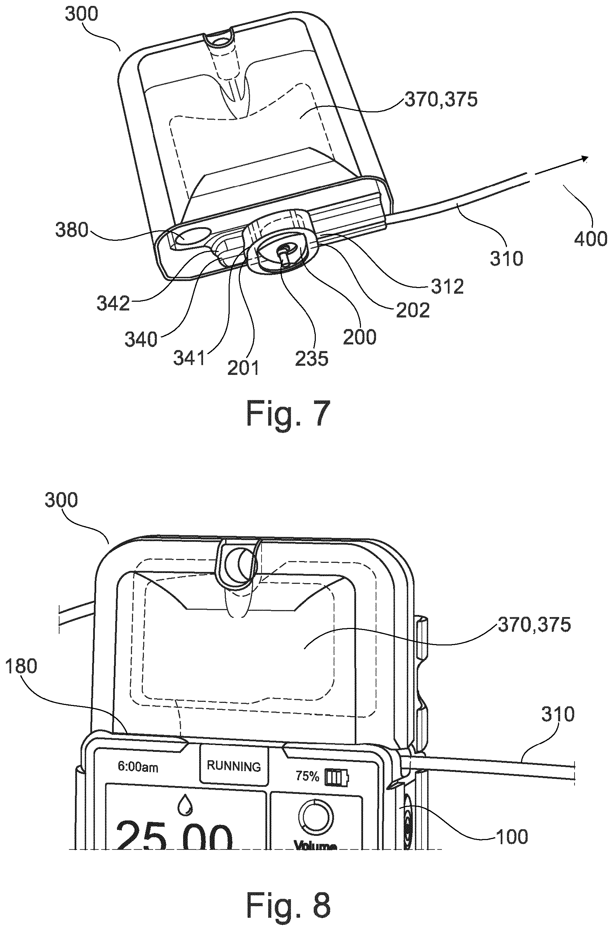

Figure 3a and Figure 3b illustrate a medicament pump driving device 100 with removed cover, so that the interior can be seen. It should be noted that this is not a usual operation condition, as usually the medicament pump driving device 100 is closed, or hermetically closed. The trochoidal medicament pump device 200 is not yet coupled to the medicament pump driving device 100. The medicament pump driving device 100 has an output driving interface 135 to which a corresponding input driving interface 235 of the trochoidal medicament pump device 200 can be coupled. For this purpose, the output driving interface 135 has a coupling 135b, which matches a corresponding coupling 235b of the trochoidal medicament pump device 100. This matching ensures that the output driving interface 135 can be coupled to the input driving interface 235 only in a limited number of defined rotational positions. In a particular embodiment, the coupling may take place in three equidistant rotational positions of the rotor, i.e., every 120°. As the trochoidal medicament pump device 200 as described above, has a triangular rotor 230, the rotational position repeats every 120°. Consequently, the relative rotational position of the rotor 230 over the output driving interface 235 is the same every 120°. The medicament pump driving device 100 has a housing 120 and formed therein a receptacle 120d. The housing is illustrated with a removed cover to illustrate the interior. The receptacle 120d may be bayed in the driving device for receiving the trochoidal medicament pump device 200, as described above. The receiving canal 121 serves for receiving a first/inlet fluid conduit, which may be releasable or fixed connected to the in-port 201. The receiving canal 122 serves for receiving a second/outlet fluid conduit, which may be releasable or fixed connected to the out-port 202. For driving the rotor 230 of the trochoidal medicament pump device 200, the medicament pump driving device 100 has an output driving interface 135, which can be driven by a drive unit 110. A control unit 140 can control the driving characteristic of the drive unit 110. As the driving characteristic of a trochoidal medicament pump device 200 is not linear, the rotational position of the rotor 230 of the trochoidal medicament pump device 200 over its pump body 220 may be signaled by a position signaling unit 250. The position signal provided by the position signaler 250 may be read out by a position signal receiving sensor 150, so that based thereon the control unit 140 may control the drive unit 110. The medicament pump driving device 100 may also include a data interface 194 for exchanging information with an external data storage or data processing unit. This may include storing dosing data of administered medicament in order to monitor the delivered dose. This may also include receiving data for controlling the drive unit 110, so that setup and maintenance of the medicament pump driving device 100 may be carried out remotely. A locking mechanism 160 as described with respect to Figures 2a and 2b may be used for the purposes as describe above. A locking mechanism on the medicament pump driving device 100 may engage a corresponding locking geometry 260 on the trochoidal medicament pump device 200. The locking mechanism may release the trochoidal medicament pump device 200 so that it can be removed from the medicament pump driving device 100 only when it is not in operation. Operation here may include breaks or pauses of application. The locking mechanism 160 may also be designed so that is can only release upon authorization e.g. from clinical personal, doctors, and/or the patient. The access cover 165 may cover the vulnerable components of the trochoidal medicament pump device 200. The medicament pump driving device may have an energy storage 170, which may be e.g., a single use battery or a rechargeable battery. A wireless charging interface 197 for charging a re-usable battery 170 may be provided within the medicament pump driving device 100. A user may operate the medicament pump driving device via a user interface 175, which may be a bi-directional user interface. Input may include a keyboard entering of data, voice recording or gesture. Output may include optical output via e.g. a screen, acoustic output via speaker or vibrating units. The medicament pump driving device 100 may also have a tag reader 180, being capable of reading a tag 380 on the trochoidal medicament pump device 100 or a reservoir 175 connected to the trochoidal medicament pump device 100, as illustrated in Figure 8. The medicament pump driving device 100 may also have a patient identification reader 185, being capable of reading an identification item of a patient, either a bio-characteristic feature like a fingerprint or an iris, or a tag attached to the patient. This may avoid a wrong medicament or a wrong dose for a particular patient.

Figure 4 illustrates a top view of a trochoidal medicament pump device 200 with removed cover according to an exemplary embodiment. Figure 4 illustrates the trochoidal medicament pump device with removed cover 220b and with removed driving pinion 235. Upon rotation of the pump rotor 230 in a planetary gear manner around the gear wheel 224, which is fixed to the pump body 220, the apexes 231 of the pump rotor 230 follow the trochoidal shape of the pump chamber 225, in particular its wall portion 227a on the right side in Figure 4. The apex lines 231a seal the pump rotor 230 over the inner wall portions and this form the suction chamber 228a and the expelling chamber 229a, respectively. The fluid to be pumped and delivered enters the pump chamber 225 over the inlet 221a and leaves the pump chamber 225 over the outlet 222a. The illustrated situation in Figure 4 is the transit where the suction chamber 228a becomes an expelling chamber 229a. It can be seen, that one of the apexes 231 has just passed the inlet 221a and thus closed the chamber and terminated the suction phase, before the ahead traveling apex 231 has not yet passed the outlet 222a and thus not yet opened the chamber for expelling the fluid. It should be noted that the apexes 231 also follow the wall sections 227b on the other side, the left side in Figure 4. However, in the illustrated embodiment of Figure 4, which corresponds to the embodiment illustrated in Figure 1, only one inlet 221a and only one outlet 222a are provided on the right lobe 226a. The other lobe 226b on the left side does not have an inlet and an outlet, so that in the respective the suction chamber 228b on the left side, a vacuum arises upon rotation. At the same time in the expelling chamber 229b on the left side the pressure arises upon rotation. As the vacuum in chamber 228b and the over pressure in chamber 229b lead to increased friction and load of the rotor 230, this vacuum and pressure, respectively should be avoided. As a further inlet and outlet should be avoided in case they are not used for pump and delivery, the pressure difference is compensated through a notch 227c, which follows along a particular section of the wall section 227b on the left side in Figure 4. The notch bypasses the sealing of the apex 231 at the inner wall portion 227b and allows the overpressure from the expelling chamber to transit and compensate the vacuum in the suction chamber 228b. Thus, the pressure difference can be compensated, and the friction and load can be reduced. The notch may be formed by a chamfer as illustrated in Figure 4. The chamfer may have rounded edges at least at the side where the apex line is in contact with the edge of the chamfer. The notch may also be realized by a groove along the inner wall portion 227b or even by a bypassing channel having one opening in the area of the suction chamber 228b and the other opening in the area of the expelling chamber 229b.

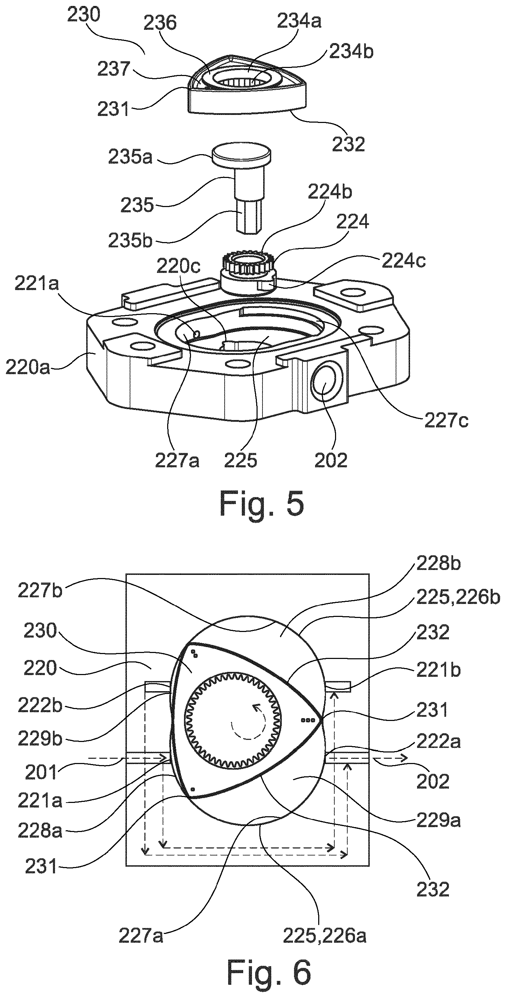

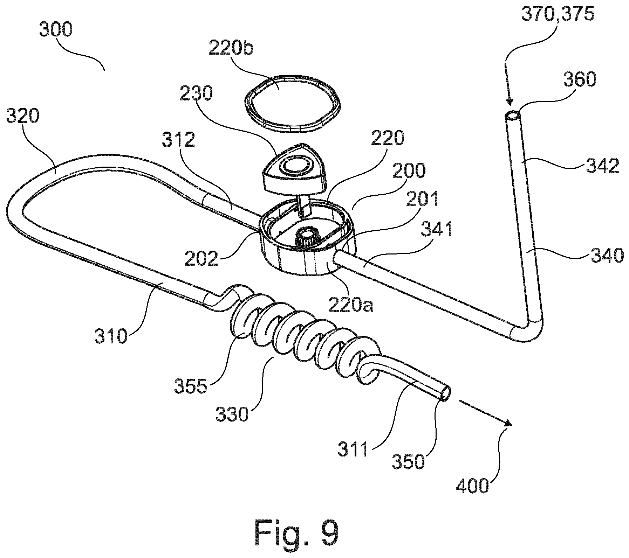

Figure 5 illustrates an exploded view of a trochoidal medicament pump device without cover 220b according to another exemplary embodiment. The position of the cover 220b is illustrated in Figure 9. It should be noted that the traveling trajectory of the pump rotor 230 is defined by the eccentric disk 235a, which is illustrated in Figure 5, and which is omitted in Figure 4 for illustrative purposes. The pump rotor 230 has a sliding or guiding surface 234a, which receives an eccentrically traveling disk 235a of a driving pinion 235, which eccentrically rotation disk 235a forces the pump rotor 230 onto an eccentric trajectory. The eccentrically traveling disk 235a and the drive coupling 235b of the driving pinion 235 may manufactured in one piece. The pump rotor 230 has also a gear section 234b, which meshes with a gear section 224b of a gear wheel 224 on the pump body 220, in particular the housing 220a, and upon eccentric traveling, forces the pump rotor 230 to rotate. In Figure 4 the pinion 235 and the eccentrically rotation disk 235a thereof being removed. The design of the driving pinion 235 can be seen in Figure 5. The eccentrically rotating disk 235a upon centric rotation of the pinion 235 and its drive coupling 235b forces the pump rotor 230 onto an eccentric path. The eccentric disk 235a is rotationally bedded over a corresponding bearing surface 234a on the rotor 230, in particular its core 236. As during eccentric rotation the gear section 234b of the pump rotor 230 remains in engagement with the gear section 224b of gear wheel 224 of the pump body, the pump rotor 230 not only eccentrically travels along an eccentric path, but also rotates, so that the apexes follow the trochoidal path of the first and second lobe 226a, 226b and the corresponding wall portions 227a, 227b. As can be seen in Figure 5, the gear wheel 224 is provided as a separate element which however has a protrusion 224c which engages a detention 220c in the pump body so that the gear wheel 224 is fixed over the pump body. The fixation can also be realized by other measures, like adhesives, positive fit connections or by providing the gear wheel 224 in one piece with the pump body 225. With respect to the remaining elements in Figure 5 it is referred to the above-described figures.

The pump rotor 230 has an over-mold 237, which has with respect to the contacting portions to the wall portions 227a, 227b of the pump chamber 225 a larger dimension than the pump chamber, so that the compressible over-mold is compressed and provides a reliable sealing. These contacting portions are the apex lines 231a of the rotor 230 and also portions of the convex curved surfaces 232a, which are in contact to the inner wall portions 227a, 227b, in particular with the transit from the first wall portion 227a of the first lobe 226a to the second wall portion 227b of the second lobe 226b.

Figure 6 illustrates a schematic build-up in a top view of a trochoidal medicament pump device according to another exemplary embodiment. The trochoidal medicament pump device 200 as illustrated in Figure 6 is similar to the device illustrated in Figure 1. In addition to what is described with respect to Figure 1, the trochoidal medicament pump device 200 of Figure 6 has not only an inlet 221a and an outlet 222a in the first lobe 226a, but also has a further inlet 221b and a further outlet 222b in the second lobe 226b. This allows using also the second lobe as a pump, which may be operated in parallel to the pump formed in the first lobe 226a. The first inlet 221a and the second inlet 221b both may be in fluid communication with the in-port 201. At the same time the first outlet 222a and the second outlet 222b may be in fluid communication with the out-port 202. Thus, both pump paths can be coupled via a common in-port 201 and a common out-port 202. The in-port 201 receives the fluid or liquid to be pumped and the out-port 202 delivers the fluid or liquid to be delivered. The in-port and the out-port may have releasable couplings or may be fixedly connected to conduits or the like. The triangular pump rotor 230 travels as describe above. The apex lines 231a and the convex curved lines 232a are in contact with wall portions 227a, 227b of the pump chamber 225. The apex lines 231a are always in contact with one of the wall portions 227a, 227b. The pump chamber 225 has two lobes 226a, 226b. Within the pump chamber 225 the pump rotor 230 rotates, so that the contact between the pump rotor 230, in particular its apex lines 231a and also the convex curved surfaces 232a, and the wall sections 227a, 227b of the lobes 226a, 226b form chambers, which rotate with the eccentrically rotating pump rotor 230 and successively are brought into communication with the first inlet 221a, the first outlet 222a. the second inlet 221b and the second outlet 222b. When in connection with the respective inlet 221a, 221b, the chamber operates as a suction chamber 228a, 228b. As the chamber upon rotation successively enlarges its volume, the chamber sucks a fluid or liquid from the in-port 201. Upon rotation, the suction chamber rotates until it leaves the respective inlet 221a, 221b. Afterwards, the chamber gets into communication with the respective outlet 222a, 222b and becomes an expelling chamber 229a, 229b. Upon further rotation, the expelling chamber 229a, 229b successively decreases its volume and thus expels the fluid through the output 222a, 222b until the volume arrives at its minimum, which may be close to zero. At the same time, the following geometry of the rotor 230 proceeds likewise, so that the next chamber becomes the expelling chamber 229a, 229b and expels the next fluid amount upon rotation of the pump rotor 230. The in-flow follows in Figure 6 the dashed lines and arrows, while the out flow follows the dashed-dotted lines in Figure 6.

As can be seen from Figure 6, the first suction chamber 228a is at a minimum volume starting the suction process, while the second suction chamber 228b is at a maximum volume having almost finished the suction process. Likewise, the first expelling chamber 229a is at a maximum volume starting the expelling process, while the second expelling chamber 229b is at a minimum volume having almost finished the expelling process. As the expelling process is not linear and has a break while an apex crosses the respective output, a single pump has a non-continuous feeding upon continuous rotation of the pump rotor 230. However, as the expelling process of the pump in the first lobe 226a is phase shifted over the expelling process in the second lobe 226b, the interruption may be compensated. So that no interruption occurs at the out-port 202. In order to avoid unintended backflow of fluid into a chamber, check valves or flow resistors (not illustrated in Figure 6) may be provided which avoid or reduce unintended back flow.

Figure 7 illustrates a trochoidal medicament pump device 200 with a connected liquid/fluid reservoir and a trochoidal medicament pump device according to an exemplary embodiment. The trochoidal medicament pump device is as described above with respect to Figures 1 to 6. The trochoidal medicament pump device 200 with a connected liquid/fluid reservoir may be designed as a giving set 300. A giving set is a device which is used for applying a medicament to a patient. Conventional giving sets are known and may include a connection to a reservoir and a connection to a patient and dosing facilities for setting a flow rate. The giving set 300 includes a trochoidal medicament pump device 200 and further has a first fluid conduit 310 for connecting the trochoidal medicament pump device 200, in particular its out-port 202 to a patient 400. Likewise, the giving set 300 has a second fluid conduit 340 for connecting the trochoidal medicament pump device 200, in particular its in-port 201 with a reservoir 370. The giving set 300 illustrated in Figure 7 has the reservoir 370 fixedly connected to the second fluid conduit 340, The first end 341 of the second conduit 340 is fixedly connected to the in-port 201 of the trochoidal medicament pump device 200 and the second end 342 of the second conduit 340 is fixedly connected to the reservoir 370. This reduces joining connections and impurities in the system. The reservoir 370 may be a pre-filled reservoir 375, so that the giving set is ready for use in combination with a trochoidal medicament pump device 200. The first conduit 310 with its first end can be connected to the patient 400, and with its second end 312 is fixedly connected to the out-port 202 of the trochoidal medicament pump device 200. The trochoidal medicament pump device 200 may be adapted to the fluid in the prefilled reservoir 375. As the reservoir usually is not refilled and disposed, also the trochoidal medicament pump device 200 is disposed and an unintended re-use can be avoided. The reservoir may be a flexible volume like a bag and with the fluid therein may be gas free, so that irrespective of the position of the reservoir and its outlets, a fluid can be taken from the reservoir. As the trochoidal medicament pump device 200 is able to suck, the reservoir may even be disposed lower than the trochoidal medicament pump device 200, i.e., with the trochoidal medicament pump device 200 on top of the reservoir 370, 375. The giving set 300 may have a readable tag 380 carrying information representative for the giving set, e.g., with respect to the tape of fluid, the volume of the reservoir, the type of the trochoidal medicament pump device 200, etc. This tag can read by a tag reader 180 on e.g., a driving device 100 for the trochoidal medicament pump device 200, as will be described with respect to Figure 8. The tag 380 may be an RFID device, or a machine-readable optical tag, but may also be an actively sending tag. The tag may also include a certain intelligence and may be connected to sensors or the like, which sense parameters on or of the giving set, so that this information can be provided upon reading the tag. The trochoidal medicament pump device 200 has a coupling to be driven by a driving device 100. The coupling may be realized by a coupling portion 235 of a driving pinion, as describes above, and may be coupled to the driving device 100 by the coupling portion 235.

Figure 8 illustrates giving set with a connected liquid/fluid reservoir and a trochoidal medicament pump device connected to driving device according to an exemplary embodiment. The giving set 300 is coupled via the above-described driving coupling 235 to the driving unit 100. The driving unit may also have a tag reading facility 180 for reading the tag 380 or communicating with the tag 380. The trochoidal medicament pump device 200 in this embodiment is received in aby of the driving unit in order to protect the trochoidal medicament pump device 200, its ports 201, 202 and also the conduits 310, 340. The giving set 300 may be locked with respect to the driving unit 100 in order to avoid unintended separation of the reservoir 370, 375 and the trochoidal medicament pump device 200 from the driving unit.

Figure 9 illustrates giving set with a fluid conduit to be connected to a patient and a fluid conduit to be connected to a liquid reservoir according to an exemplary embodiment. Figure 9 illustrates the implementation of a trochoidal medicament pump device as described above with respect to Figures 1 to 6. The giving set 300 includes the trochoidal medicament pump device 200 and further has a first fluid conduit 310 for connecting the trochoidal medicament pump device 200, in particular its out-port 202 to a patient 400. Likewise, the giving set 300 has a second fluid conduit 340 for connecting the trochoidal medicament pump device 200. For this purpose, the second conduit 340 has a first end 341, which is connected to the trochoidal medicament pump device 200, in particular its in-port 201. A second end 342 of the second conduit 340 may be connected to a reservoir 370, 375. This can be done via a connector 360. The reservoir 370, 375 may also be fixedly connected as described with respect to figure 7 above. The giving set illustrated in Figure 9 for the first conduit 310 has an upstream section 320 and a downstream section 330. In the upstream section 320 the second end 312 of the first conduit 310 is fixedly connected to the out-port 202 of the trochoidal medicament pump device 200. In the downstream section 330 the first end 311 of the first conduit 310 is connectable to a patient 400, e.g., via a Luer lock device 350. The downstream section 330 in this embodiment has a higher flexibility than the upstream section, which means that it can be easier bended. This is achieved in the illustrated embodiment by providing a helical winding 335, which elongates the flow length of the conduit and therefore allows higher flexibility, however without elongated dimensions, as the helical winding 335 is not longer than a comparable other section and does not increase the physical distance from the reservoir to the patient. The helical winding may not only compensate bending forces traverse to the longitudinal extension, but also forces along the longitudinal extension of the first conduit, as the helical winding may act like a spring.

### Reference numbers

- 100: driving device for driving the trochoidal medicament pump device
- 110: drive unit
- 120: housing of driving device
- 120d: receptacle bayed in driving device for receiving trochoidal medicament pump device
- 121: receiving canal for first/inlet fluid conduit
- 122: receiving canal for second/outlet fluid conduit
- 135: output driving interface
- 135b: coupling of output driving interface of driving device
- 140: control unit of driving device
- 150: position receiving sensor of driving device
- 160: locking mechanism of driving device
- 165: access cover of driving device
- 170: energy storage, (rechargeable) battery of driving device
- 175: user interface, keypad, of driving device
- 180: tag reader of driving device
- 185: patient identification reader of driving device
- 194: dosing data interface of driving device
- 197: wireless charging interface of driving device
- 200: trochoidal medicament pump device
- 201: in-port of trochoidal medicament pump device
- 202: out-port of trochoidal medicament pump device
- 220: pump body
- 220a: housing of pump body
- 220b: cover of pump body
- 220c: locking notch of pump housing / pump body
- 220d: outer geometry of pump body
- 221a: first inlet of pump chamber
- 221b: second inlet of pump chamber
- 222a: first outlet of pump chamber
- 222b: second outlet of pump chamber
- 224: gear wheel
- 224b: gear section of gear wheel
- 224c: locking protrusion of gear wheel
- 225: pump chamber
- 226a: first lobe of pump chamber
- 226b: second lobe of pump chamber
- 227a: first inner wall portion
- 227b: second inner wall portion
- 227c: notch
- 228a: first suction volume
- 228b: second suction volume
- 229a: first expelling volume
- 229b: second expelling volume
- 230: pump rotor
- 231: apex
- 231a: apex line / apex sealing line of pump rotor
- 232: convex curved line /convex curved sealing line of pump rotor
- 232a: convex curved surface
- 234a: sliding / guiding surface on pump rotor / rigid rotor core of pump rotor
- 234b: gear section on pump rotor / rigid rotor core of pump rotor
- 235: input driving interface, drive pinion
- 235a: eccentric sliding disk of drive pinion
- 235b: coupling of drive pinion/input driving interface, drive coupling of drive pinion
- 236: (rigid) rotor core of pump rotor
- 237: (compressible) over-mold of pump rotor
- 250: position signaler for rotational position of pump rotor over pump housing
- 260: locking receptacle of trochoidal medicament pump device
- 300: giving set
- 310: first fluid conduit
- 311: first end of first fluid conduit
- 312: second end of first fluid conduit
- 320: upstream section of first fluid conduit
- 330: (flexible) downstream section of first fluid conduit
- 335: (flexible) helical winding of first fluid conduit
- 340: second fluid conduit
- 341: first end of second fluid conduit
- 342: second end of second fluid conduit
- 350: Luer lock (at (first) end of first fluid conduit)
- 360: connector at (second) end of second fluid conduit
- 370: fluid reservoir
- 375: pre-filled fluid reservoir
- 380: tag on (pre-filled) fluid reservoir
- 400: patient
- 485: patient identifier

## Claims

1. A medicament administering system comprising
a medicament pump driving device (100) to be coupled to a trochoidal medicament pump device (200) for controlled driving of the trochoidal medicament pump device (200); and
the trochoidal medicament pump device (200);
wherein the medicament pump driving device (100) comprises:
a housing (120);
a receptacle (120d) bayed in said housing (120);
an output driving interface (135);
wherein the trochoidal medicament pump device (200) comprises:
a pump body (220) with an outer geometry (220d) being adapted for being received in the receptacle (120d) bayed in said housing (120) of said medicament pump driving device (100);
an in-port (201) for entering a medicament to be administered;
an out port (202) for administering a medicament;
a pump rotor (230) having a convex triangular cylinder shape and having an eccentric trajectory upon rotation within the pump body;
an input driving interface (235) coupled to the pump rotor (230) and being adapted for releasable coupling to the output driving interface (135) of said medicament pump driving device (100),
wherein the pump rotor (230) has three equidistant apexes (231), each two thereof are connected with a convex curved line (232);
wherein the pump body (220) has a pump chamber (225);
wherein the pump chamber (225) has a first lobe (226a) forming a first inner wall portion (227a) of a pump volume of the pump chamber (225) with a first inlet (221a) in fluid communication with the in-port (201) and a first outlet (222a) in fluid communication with the out-port (202) with the pump rotor (230) eccentrically rotating so that at least three distinct portions (231, 232) of the rotor (230) are in permanent sealing contact with the first inner wall portion (227a) of the first lobe (226a) thus forming upon rotation a first suction volume (228a) in fluid communication to the first inlet (221a) and a first expelling volume (229a) in fluid communication to the first outlet (222a),
wherein the receptacle (120d) bayed in said housing is adapted for releasable receiving of said pump body (220) of said trochoidal pump device (200) to be connected to the medicament pump driving device (100);
wherein the output driving interface (135) is adapted for releasable receiving of said input driving interface (235) of said trochoidal medicament pump device (200) for driving said pump rotor (230);
wherein the medicament pump driving device (100) further comprises:
a drive unit (110) arranged within the housing (120) for rotatable driving via the output driving interface (135) said pump rotor (230) of said trochoidal medicament pump device (200);
a control unit (140) for controlling the drive unit (110) to drive the output driving interface (135) based on a provided dosing signal;
wherein for operation the trochoidal medicament pump device (200) with its pump body (220) is bayed in a receptacle (120d) in the housing of the medicament pump driving device (100) for releasable receiving the pump body (220);
wherein an outer shape (220d) of the pump body (220) matches the receptacle (120d) in that the pump body (220) upon reception within the receptacle (120d) is rotationally fixed to the housing (120) of the medicament pump driving device (100).

2. The medicament administering system of claim 1, wherein the output driving interface (135) comprises a coupling (135b) having an engaging geometry for receiving a corresponding counter coupling (235b) of said input driving interface (235) for coupling in a number of defined rotational positions of said pump rotor (230) of said trochoidal medical pump device (200),

3. The medicament administering system of any of claims 1 and 2, wherein the medicament pump driving device (100) comprises a position receiving sensor (150) for receiving a rotational position of said pump rotor (230).

4. The medicament administering system of any of claims 1 to 3, wherein the medicament pump driving device (100) comprises a locking mechanism (160) being adapted for locking said trochoidal medicament pump device (200) into the pump driving device during a driving operation.

5. The medicament administering system of any of claims 1 to 4, wherein the medicament pump driving device (100) comprises an access cover (165) being adapted for securely covering said trochoidal medicament pump device (200) and releasing a driving operation.

6. The medicament administering system of any of claims 1 to 5, wherein the medicament pump driving device (100) comprises a dosing data interface (194) being operatively connected to the control unit (140) for providing dosing data to and from the medicament pump driving device (100).

7. The medicament administering system of any of claims 1 to 6, wherein the medicament pump driving device (100) comprises a tag reader (180) being adapted for reading a tag on a medicament reservoir being fixedly connected to said trochoidal medicament pump device (200).

8. The medicament administering system of any of claims 1 to 7, wherein the medicament pump driving device (100) comprises at least one of a patient identification reader (185) being adapted for reading a patient identifier (485) and releasing the operation of the drive unit (110) upon a patient identification, and a healthcare professional identification reader (185) being adapted for reading a healthcare professional identifier (485) and tracking the operation of the drive unit (110) upon a healthcare professional identification.

9. The medicament administering system of any of claims 1 to 8, wherein the pump chamber (225) has a second lobe (226b) symmetrical to the first lobe (226a), a second suction volume (228b) and a second expelling volume (229b) wherein the second lobe (226b) has formed therein a pressure-bypass between the second suction volume (228b) and a second expelling volume (229b).

10. The medicament administering system of any of claims 1 to 9, wherein the trochoidal medicament pump device (200) comprises a position signaler (250) for signaling a rotational position of the pump rotor (230) within the pump body (220).

11. A giving set (300) in combination with the medicament administering system of any of claims 1 to 10, the giving set (300) configured to provide a medicament from a reservoir to a patient (400), the giving set (300) comprising:
the trochoidal medicament pump device (200) and
a first fluid conduit (310) being with one end (312) fixedly connected to the out-port (202) and with the other end (311) fixedly connected to a Luer-lock (350).

12. The giving set of claim 11, wherein the first fluid conduit (310) is made of a flexible tube of substantially constant diameter and wall thickness, wherein a flexible downstream section (330) is formed by a pre-formed helical winding (335) of the flexible tube, the geometry thereof providing higher flexibility than a non-helical portion.

13. The giving set of any of claims 11 and 12, wherein the giving set (300) further comprises a second fluid conduit (340) being with one end (341) fixedly connected to the in-port (201) and with the other end (342) fixedly connected to a pre-filled fluid reservoir (375).

14. The giving set of any of claims 11 to 13, wherein the giving set (300) further comprises a tag (380) representative for a liquid in the pre-filled reservoir (375) and being adapted to be read out by a tag reader (180) of said medicament pump driving device (100).

## Patentansprüche

1. Ein Medikamentenverabreichungssystem, aufweisend:
eine Medikamentenpumpenantriebsvorrichtung (100), die mit einer trochoidalen Medikamentenpumpenvorrichtung (200) zum gesteuerten Antrieb der trochoidalen Medikamentenpumpenvorrichtung (200) zu verbinden ist;
die trochoidale Medikamentenpumpenvorrichtung (200);
wobei die Medikamentenpumpenantriebsvorrichtung (100) umfasst:
ein Gehäuse (120);
eine in das Gehäuse (120) eingelassene Aufnahme (120d);
eine Ausgangsantriebsschnittstelle (135);
wobei die trochoidale Medikamentenpumpenvorrichtung (200) umfasst:
einen Pumpenkörper (220) mit einer äußeren Geometrie (220d), die dazu ausgebildet ist, in der Aufnahme (120d) aufgenommen zu werden, die in das Gehäuse (120) der Medikamentenpumpenantriebsvorrichtung (100) eingelassen ist;
einen Einlass (201) zum Einführen eines zu verabreichenden Medikaments;
einen Auslass (202) zur Verabreichung eines Medikaments;
einen Pumpenrotor (230) mit einer konvexen dreieckigen Zylinderform und einer exzentrischen Trajektorie bei der Rotation innerhalb des Pumpenkörpers;
eine Eingangsantriebsschnittstelle (235), die mit dem Pumpenrotor (230) gekoppelt ist und für eine lösbare Kopplung mit der Ausgangsantriebsschnittstelle (135) der Medikamentenpumpenantriebsvorrichtung (100) ausgelegt ist,
wobei der Pumpenrotor (230) drei äquidistante Scheitelpunkte (231) aufweist, von denen jeweils zwei mit einer konvex gekrümmten Linie (232) verbunden sind;
wobei der Pumpenkörper (220) eine Pumpkammer (225) aufweist;
wobei die Pumpenkammer (225) eine erste Keule (226a) aufweist, die einen ersten Innenwandabschnitt (227a) eines Pumpvolumens der Pumpenkammer (225) mit einem ersten Einlass (221a) in Fluidverbindung mit dem Einlass (201) und einem ersten Auslass (222a) in Fluidverbindung mit dem Auslass (202) bildet, wobei sich der Pumpenrotor (230) exzentrisch dreht, so dass mindestens drei verschiedene Abschnitte (231, 232) des Rotors (230) in ständigem Dichtungskontakt mit dem ersten Innenwandabschnitt (227a) der ersten Keule (226a) stehen, wodurch bei der Rotation ein erstes Ansaugvolumen (228a) in Fluidverbindung mit dem ersten Einlass (221a) und ein erstes Ausstoßvolumen (229a) in Fluidverbindung mit dem ersten Auslass (222a) gebildet wird,
wobei die in das Gehäuse eingelassene Aufnahme (120d) zur lösbaren Aufnahme des Pumpenkörpers (220) der mit der Medikamentenpumpenantriebsvorrichtung (100) zu verbindenden trochoidalen Pumpenvorrichtung (200) ausgebildet ist;
wobei die Ausgangsantriebsschnittstelle (135) zur lösbaren Aufnahme der Eingangs-Antriebsschnittstelle (235) der trochoidalen Medikamentenpumpenvorrichtung (200) zum Antrieb des Pumpenrotors (230) ausgebildet ist;
wobei die Medikamentenpumpenantriebsvorrichtung (100) ferner umfasst:
eine Antriebseinheit (110), die innerhalb des Gehäuses (120) angeordnet ist, um über die Ausgangsantriebsschnittstelle (135) den Pumpenrotor (230) der trochoidalen Medikamentenpumpenvorrichtung (200) drehbar anzutreiben;
eine Steuereinheit (140) zum Steuern der Antriebseinheit (110), um die Ausgangsantriebsschnittstelle (135) basierend auf einem bereitgestellten Dosiersignal anzutreiben;
wobei für den Betrieb die trochoidale Medikamentenpumpenvorrichtung (200) mit ihrem Pumpenkörper (220) in eine Aufnahme (120d) im Gehäuse der Medikamentenpumpenantriebsvorrichtung (100) zur lösbaren Aufnahme des Pumpenkörpers (220) eingelassen ist;
wobei eine äußere Form (220d) des Pumpenkörpers (220) an die Aufnahme (120d) angepasst ist, so dass der der Pumpenkörper (220) bei Aufnahme in der Aufnahme (120d) drehfest mit dem Gehäuse (120) der Medikamentenpumpenantriebsvorrichtung (100) verbunden ist.

2. Medikamentenverabreichungssystem nach Anspruch 1, wobei die Ausgangsantriebsschnittstelle (135) eine Kupplung (135b) mit einer Eingriffsgeometrie zur Aufnahme einer entsprechenden Gegenkupplung (235b) der Eingangsantriebsschnittstelle (235) zur Kopplung in einer Anzahl von definierten Rotationspositionen des Pumpenrotors (230) der trochoidalen medizinischen Pumpenvorrichtung (200) umfasst.

3. Das Medikamentenverabreichungssystem nach einem der Ansprüche 1 und 2, wobei die Medikamentenpumpenantriebsvorrichtung (100) einen Positionsempfangssensor (150) zum Empfangen einer Rotationsposition des Pumpenrotors (230) umfasst.

4. Medikamentenverabreichungssystem nach einem der Ansprüche 1 bis 3, wobei die Medikamentenpumpen-Antriebsvorrichtung (100) einen Verriegelungsmechanismus (160) umfasst, der zum Verriegeln der trochoidalen Medikamentenpumpenvorrichtung (200) in der Pumpenantriebsvorrichtung während eines Antriebsvorgangs ausgebildet ist.

5. Medikamentenverabreichungssystem nach einem der Ansprüche 1 bis 4, wobei die Medikamentenpumpen-Antriebsvorrichtung (100) eine Zugangsabdeckung (165) umfasst, die zum sicheren Abdecken der trochoidalen Medikamentenpumpenvorrichtung (200) und zum Freigeben eines Antriebsvorgangs ausgebildet ist.

6. Das Medikamentenverabreichungssystem nach einem der Ansprüche 1 bis 5, wobei die Medikamentenpumpenantriebsvorrichtung (100) eine Dosierdatenschnittstelle (194) umfasst, die funktionell mit der Steuereinheit (140) verbunden ist, um Dosierdaten an die und von der Medikamentenpumpenantriebsvorrichtung (100) bereitzustellen.

7. Das Medikamentenverabreichungssystem nach einem der Ansprüche 1 bis 6, wobei die Medikamentenpumpenantriebsvorrichtung (100) ein Etikett-Lesegerät (180) umfasst, das zum Lesen eines Etiketts an einem Medikamentenreservoir, das fest mit der trochoidalen Medikamentenpumpenvorrichtung (200) verbunden ist, ausgebildet ist.

8. Medikamentenverabreichungssystem nach einem der Ansprüche 1 bis 7, wobei die Medikamentenpumpenantriebsvorrichtung (100) mindestens einen Patientenidentifikationsleser (185), der zum Lesen einer Patientenidentifikation (485) und zur Freigabe des Betriebs der Antriebseinheit (110) bei einer Patientenidentifikation ausgebildet ist, und einen Identifikationsleser für medizinisches Fachpersonal (185), der zum Lesen einer Identifikation für medizinisches Fachpersonal (485) und zur Verfolgung des Betriebs der Antriebseinheit (110) bei einer Identifikation für medizinisches Fachpersonal ausgebildet ist, umfasst.

9. Medikamentenverabreichungssystem nach einem der Ansprüche 1 bis 8, wobei die Pumpenkammer (225) eine zweite, zur ersten Keule (226a) symmetrische Keule (226b), ein zweites Ansaugvolumen (228b) und ein zweites Ausstoßvolumen (229b) aufweist, wobei die zweite Keule (226b) in sich einen Druck-Bypass zwischen dem zweiten Ansaugvolumen (228b) und einem zweiten Ausstoßvolumen (229b) ausgebildet hat.

10. Medikamentenverabreichungssystem nach einem der Ansprüche 1 bis 9, wobei die trochoidale Medikamentenpumpenvorrichtung (200) einen Positionssignalgeber (250) zur Signalisierung einer Rotationsposition des Pumpenrotors (230) innerhalb des Pumpenkörpers (220) umfasst.

11. Verabreichungsset (300) in Kombination mit dem Medikamentenverabreichungssystem nach einem der Ansprüche 1 bis 10, wobei das Verabreichungsset (300) so konfiguriert ist, dass es einem Patienten (400) ein Medikament aus einem Reservoir zur Verfügung stellt, wobei das Verabreichungsset (300) Folgendes umfasst:
die trochoidale Medikamentenpumpenvorrichtung (200) und
eine erste Flüssigkeitsleitung (310), die mit einem Ende (312) fest mit dem Auslass (202) und mit dem anderen Ende (311) fest mit einem Luer-Lock (350) verbunden ist.

12. Verabreichungsset nach Anspruch 11, wobei die erste Flüssigkeitsleitung (310) aus einem flexiblen Schlauch mit im Wesentlichen konstantem Durchmesser und konstanter Wandstärke hergestellt ist, wobei ein flexibler stromabwärtiger Abschnitt (330) durch eine vorgeformte schraubenförmige Wicklung (335) des flexiblen Schlauchs gebildet wird, wobei dessen Geometrie eine höhere Flexibilität als ein nicht schraubenförmiger Abschnitt bereitstellt.

13. Verabreichungsset nach einem der Ansprüche 11 und 12, wobei der Verabreichungsset (300) ferner eine zweite Flüssigkeitsleitung (340) umfasst, die mit einem Ende (341) fest mit dem Einlass (201) und mit dem anderen Ende (342) fest mit einem vorgefüllten Fluidreservoir (375) verbunden ist.

14. Verabreichungsset nach einem der Ansprüche 11 bis 13, wobei das Verabreichungsset (300) ferner ein Etikett (380) umfasst, das eine Flüssigkeit in dem vorgefüllten Reservoir (375) repräsentiert und derart eingerichtet ist, dass es von einem Etikettleser (180) der Medikamentenpumpenantriebsvorrichtung (100) ausgelesen werden kann.

## Revendications

1. Système d'administration de médicament comprenant
un dispositif d'entraînement de pompe à médicament (100) destiné à être raccordé à un dispositif de pompe à médicament trochoïdal (200) pour l'entraînement contrôlé du dispositif de pompe à médicament trochoïdal (200) ; et
le dispositif de pompe à médicament trochoïdal (200) ;
dans lequel le dispositif d'entraînement de pompe à médicament (100) comprend :
un boîtier (120) ;
un réceptacle (120d) maintenu dans ledit boîtier (120) ;
une interface d'entraînement de sortie (135) ;
dans lequel le dispositif de pompe à médicament trochoïdal (200) comprend :
un corps de pompe (220) comportant une géométrie externe (220d) adaptée pour être reçue dans le réceptacle (120d) maintenu dans ledit boîtier (120) dudit dispositif d'entraînement de pompe à médicament (100) ;
un port d'entrée (201) pour introduire un médicament devant être administré ;
un port de sortie (202) pour administrer un médicament ;
un rotor de pompe (230) ayant une forme cylindrique triangulaire convexe et ayant une trajectoire excentrique lors de la rotation dans le corps de pompe ;
une interface d'entraînement d'entrée (235) raccordée au rotor de pompe (230) et adaptée pour le raccordement amovible à l'interface d'entraînement de sortie (135) dudit dispositif d'entraînement de pompe à médicament (100),
dans lequel le rotor de pompe (230) possède trois sommets équidistants (231), chaque deux d'entre eux sont reliés avec une ligne de courbure convexe (232) ;
dans lequel le corps de pompe (220) possède une chambre de pompe (225) ;
dans lequel la chambre de pompe (225) possède un premier lobe (226a) formant une première portion de paroi interne (227a) d'un volume de pompe de la chambre de pompe (225) avec une première entrée (221a) en communication fluidique avec le port d'entrée (201) et une première sortie (222a) en communication fluidique avec le port de sortie (202), le rotor de pompe (230) tournant excentriquement de sorte qu'au moins trois portions distinctes (231,232) du rotor (230) sont en contact étanche permanent avec la première portion de paroi interne (227a) du premier lobe (226a) formant ainsi lors de la rotation un premier volume d'aspiration (228a) en communication fluidique vers la première entrée (221a) et un premier volume d'expulsion (229a) en communication fluidique vers la première sortie (222a),
dans lequel le réceptacle (120d) maintenu dans ledit boîtier est adapté pour recevoir de manière amovible ledit corps de pompe (220) dudit dispositif de pompe trochoïdale (200) destiné à être relié au dispositif d'entraînement de pompe à médicament (100) ;
dans lequel l'interface d'entraînement de sortie (135) est adaptée pour recevoir de manière amovible ladite interface d'entraînement d'entrée (235) dudit dispositif de pompe à médicament trochoïdal (200) pour entraîner ledit rotor de pompe (230) ;
dans lequel le dispositif d'entraînement de pompe à médicament (100) comprend en outre :
une unité d'entraînement (110) disposée à l'intérieur du boîtier (120) pour entraîner en rotation via l'interface d'entraînement de sortie (135) ledit rotor de pompe (230) dudit dispositif de pompe à médicament trochoïdal (200) ;
une unité de commande (140) pour commander l'unité d'entraînement (110) à entraîner l'interface d'entraînement de sortie (135) sur la base d'un signal de dosage fourni ;
dans lequel, pour le fonctionnement, le dispositif de pompe à médicament trochoïdal (200) avec son corps de pompe (220) est maintenu dans un réceptacle (120d) dans le boîtier du dispositif d'entraînement de pompe à médicament (100) pour recevoir de manière amovible le corps de pompe (220) ;
dans lequel une forme extérieure (220d) du corps de pompe (220) s'adapte au réceptacle (120d) de sorte que le corps de pompe (220) lors de la réception dans le réceptacle (120d) est solidaire en rotation du boîtier (120) du dispositif d'entraînement de pompe à médicament (100).

2. Système d'administration de médicament selon la revendication 1, dans lequel l'interface d'entraînement de sortie (135) comprend un raccord (135b) ayant une géométrie d'engagement pour recevoir un contre-raccord correspondant (235b) de ladite interface d'entraînement d'entrée (235) pour le raccordement dans une nombre défini de positions rotatives dudit rotor de pompe (230) dudit dispositif de pompe à médicament trochoïdale (200).

3. Système d'administration de médicament selon l'une quelconque des revendications 1 et 2, dans lequel le dispositif d'entraînement de pompe à médicament (100) comprend un capteur de réception de position (150) pour recevoir une position rotative dudit rotor de pompe (230).

4. Système d'administration de médicament selon l'une quelconque des revendications 1 à 3, dans lequel le dispositif d'entraînement de pompe à médicament (100) comprend un mécanisme de verrouillage (160) adapté pour verrouiller ledit dispositif de pompe à médicament trochoïdal (200) dans le dispositif d'entraînement de pompe pendant une opération d'entraînement.

5. Système d'administration de médicament selon l'une quelconque des revendications 1 à 4, dans lequel le dispositif d'entraînement de pompe à médicament (100) comprend un couvercle d'accès (165) adapté pour recouvrir fermement ledit dispositif de pompe à médicament trochoïdal (200) et à déclencher une opération d'entraînement.

6. Système d'administration de médicament selon l'une quelconque des revendications 1 à 5, dans lequel le dispositif d'entraînement de pompe à médicament (100) comprend une interface de données de dosage (194) reliée de façon opérationnelle à l'unité de commande (140) afin de fournir des données de dosage à destination et provenant du dispositif d'entraînement de pompe à médicament (100).

7. Système d'administration de médicament selon l'une quelconque des revendications 1 à 6, dans lequel le dispositif d'entraînement de pompe à médicament (100) comprend un lecteur d'étiquettes (180) adapté pour lire une étiquette sur un réservoir à médicament relié solidairement audit dispositif de pompe à médicament trochoïdal (200).

8. Système d'administration de médicament selon l'une quelconque des revendications 1 à 7, dans lequel le dispositif d'entraînement de pompe à médicament (100) comprend au moins un d'un lecteur d'identification de patient (185) adapté pour lire un identifiant de patient (485) et pour déclencher l'opération de l'unité d'entraînement (110) lors de l'identification d'un patient, et un lecteur d'identification d'un professionnel de santé (185) adapté pour lire un identifiant de professionnel de santé (485) et pour suivre l'opération de l'unité d'entraînement (110) lors de l'identification d'un professionnel de santé.

9. Système d'administration de médicament selon l'une quelconque des revendications 1 à 8, dans lequel la chambre de pompe (225) possède un deuxième lobe (226b) symétrique au premier lobe (226a), un deuxième volume d'aspiration (228b) et un deuxième volume d'expulsion (229b) dans lequel le deuxième lobe (226b) a formé une dérivation en pression entre le deuxième volume d'aspiration (228b) et un deuxième volume d'expulsion (229b).

10. Système d'administration de médicament selon l'une quelconque des revendications 1 à 9, dans lequel le dispositif de pompe à médicament trochoïdal (200) comprend un signaleur de position (250) pour signaler une position rotative du rotor de pompe (230) dans le corps de pompe (220).

11. Kit d'administration (300) en combinaison avec le système d'administration de médicament selon l'une quelconque des revendications 1 à 10, le kit d'administration (300) étant conçu de manière à fournir un médicament à partir d'un réservoir vers un patient (400), le kit d'administration comprenant :
le dispositif de pompe à médicament trochoïdal (200) et
un premier conduit de fluide (310), dont une extrémité (312) est reliée solidairement au port de sortie (202) et l'autre extrémité (311) est reliée solidairement à un Luer-lock (350).

12. Kit d'administration selon la revendication 11,
dans lequel le premier conduit de fluide (310) est composé d'un tuyau flexible d'un diamètre et d'une épaisseur de paroi sensiblement constants, dans lequel une section flexible en aval (330) est formée par un enroulement hélicoïdal préformé (335) du tuyau flexible, la géométrie de celui-ci fournissant une flexibilité plus importante qu'une portion non-hélicoïdale.

13. Kit d'administration selon l'une quelconque des revendications 11 et 12,
ledit kit (300) comprenant en outre un deuxième conduit de fluide (340), dont une extrémité (341) est reliée solidairement au port d'entrée (201) et l'autre extrémité (342) est reliée solidairement à un réservoir de fluide prérempli (375).

14. Kit d'administration selon l'une quelconque des revendications 11 à 13,
ledit kit d'administration (300) comprenant en outre une étiquette (380) représentative pour un liquide dans le réservoir prérempli (375) et adaptée pour être lue par un lecteur d'étiquettes (180) dudit dispositif d'entraînement de pompe à médicament (100).
